# FASCICULE DE BREVET EUROPEEN

(11) **EP 0 487 382 B1**
(45) Date de publication et mention de la délivrance du brevet: **05.06.1996**
(21) Numéro de dépôt: 91403077.0
(22) Date de dépôt: 15.11.1991
(51) Int. Cl.: A61K 31/40

(54) **Application du buflomédil pour la protection du myocarde lors d'une angioplastie des coronaires**
Verwendung von Buflomedil zum Schutz des Herzmuskels bei Koronarangioplasie
Use of buflomedil for the protection of the myocardium by coronary angioplasty

(30) Priorité: 22.11.1990 FR 9014584
(43) Date de publication de la demande: 27.05.1992
(73) Titulaire: LABORATOIRE L. LAFON, F-94701 Maisons Alfort (FR)
(72) Inventeur: Bergmann, Steven R., Webster Groves, Missouri 63119 (US)
(74) Mandataire: Le Guen, Gérard

(56) Documents cités:
- WIENER MEDIZINISCHE WOCHENSCHRIFT, vol. 136, no. spéc., 1986, pages 17-24, Wien, AT; M. LESCHKE et al.: "Hämorheologisch-therapeutische Anwendungsmöglichkeiten bei der koronaren Herzerkrankung"
- ARZNEIMITTEL FORSCHUNG, vol. 39, no. 6, 1989, pages 670-677, DE; K. KUSHIKU et al.: "Cardiovascular actions of buflomedil and possible mechanisms involved"
- INTERNATIONAL ANGIOLOGY, vol. 4, no. 3, juillet-septembre 1985, pages 275-283; G. TASSI et al.: "Microcirculation in the elderly"
- MINERVA CARDIOANGIOLOGICA, vol. 36, no. 9, septembre 1988, pages 451-455, IT; M. ROMANO et al.: "Insufficienza coronarica acuta grave in corso di terapia per via endovenosa con buflomedil cloridrato"
- BLOOD VESSELS, vol. 28, suppl., 23 octobre 1991, pages 8-14, CH; D. NOLTE et al.: "Reduction of postischemic reperfusion injury by the vasoactive drug buflomedil"
- BIBL. CARDIOL., no. 38, 1984, pages 209-221, CH; P.M. VANHOUTTE: "Cardiovascular pharmacology of buflomedil"
- ANGIOLOGY, vol. 32, no. 10, octobre 1981, pages 663-675; A. DUBOURG et al.: "An experimental overview of a new vasoactive drug: buflomedil HCl"
- "The Merck Manual", 15 édition, 1987, pages 478-484, eds. R. Berkow et al., Merck & Co., Inc., Rahway, NJ, US

## Description

La présente invention concerne une nouvelle application thérapeutique du buflomédil.

Le buflomédil ou 4-(1-pyrrolidinyl)-1-(2,4,6-triméthoxyphényl)-1-butanone (décrit notamment dans FR-A-2 134 218) répond à la formule : 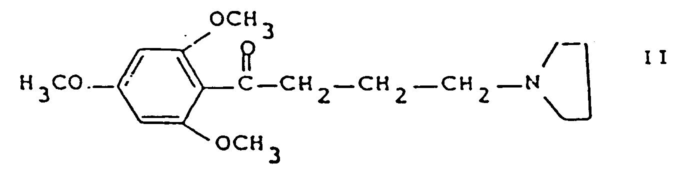
Le buflomédil sous forme de chlorhydrate est utilisé chez l'homme pour le traitement des maladies vasculaires périphériques et notamment pour le traitement des insuffisances artérielles périphériques.

Un article de Leschke et al. dans Wiener Medizinische Wochenschrift, vol. 136, n° spec. 1986, p. 17-24 indique que le buflomédil pourrait être utilisé comme traitement complémentaire dans le cas d'angor sévère.

Par ailleurs, The Merck Manual, 1987, p. 478, fait état de l'utilisation d'anticalciques dans le traitement d'angor et un article de Kushiku dans Arzneimittel Forschung, vol. 39, n° 6, 1989, p. 670, a émis l'hypothèse d'une activité anticalcique du buflomédil.

Enfin, un article de Nolte et ai. dans Blood Vessels, vol. 28, 1991, p. 8, fait état d'une amélioration par le buflomédil lors d'une reperfusion postischémique.

La présente invention a pour objet l'utilisation du buflomédil ou de ses sels pharmaceutiquement acceptables pour la fabrication d'un médicament destiné à protéger le myocarde lors d'une angioplastie des coronaires.

Dans cette application, les composés peuvent être administrés par voie orale ou parentérale et de préférence par voie intravasculaire, notamment intracoronaire, par exemple par perfusion.

La présente invention vise donc plus spécifiquement l'utilisation du buflomédil ou de ses sels pharmaceutiquement acceptables pour la fabrication de compositions administrables par voie intraveineuse, par exemple par perfusion.

Pour la fabrication des médicaments on peut utiliser tous les excipients et véhicules classiquement utilisés pour la fabrication de tels médicaments.

Ainsi notamment pour l'administration par voie intraveineuse, le composé, notamment sous forme d'un sel pharmaceutiquement acceptable, peut être dissous dans une solution isotonique pour injection ou perfusion.

La dose journalière par voie intraveineuse peut être de 3 à 6 mg/kg. Par voie orale, la dose journalière peut être de 7 à 10 mg/kg.

Dans la présente invention, on désigne par "sels pharmaceutiquement acceptables" les sels d'addition avec des acides qui donnent les propriétés biologiques du buflomédil sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; les sels métalliques acides, tels que l'orthophosphate disodique et le sulfate monopotassique, et des acides organiques, tels que l'acide formique, l'acide acétique, l'acide propionique, l'acide glycolique, l'acide oxalique, l'acide fumarique, l'acide lactique, l'acide succinique, l'acide tartrique, l'acide malique et l'acide pamoïque.

La présente invention trouve une application particulièrement intéressante dans les angioplasties des coronaires. En effet, les ballonnets utilisés dans cette intervention provoquent une ischémie et il convient de protéger le myocarde.

Les essais suivants mettent en évidence la nouvelle application du buflomédil.
1 - On a réalisé des essais comparatifs chez des chiens auxquels on a administré par voie intraveineuse en 15 minutes :
   - soit du buflomédil (chlorhydrate) à la dose de 10 mg/kg ;
   - soit un placebo.

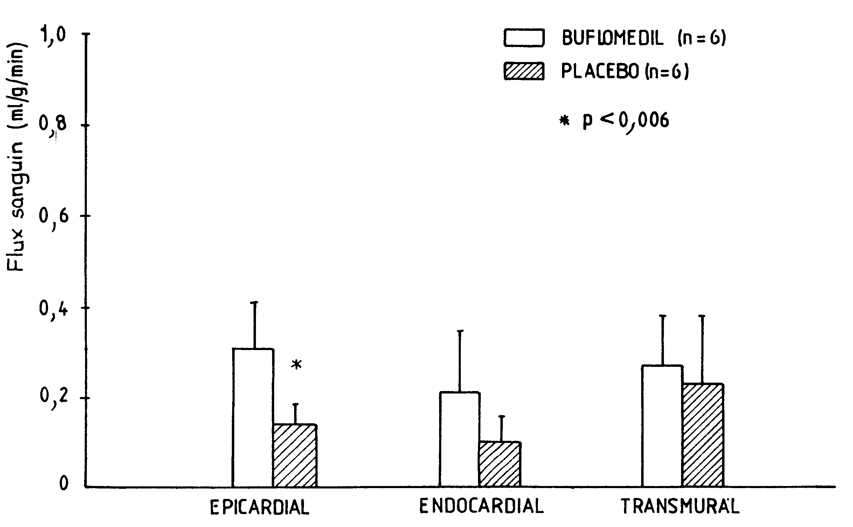

   Le protocole opératoire était le suivant : les chiens ont reçu une prémédication consistant en 1,0 mg par kg de sulfate de morphine après une nuit de diète ainsi que 325 mg d'aspirine par voie orale.
   Les chiens ont été anesthésiés avec 12,5 mg/kg de thiopental et 72 mg/kg de chloralose par voie intraveineuse. On met en place un cathéter au niveau de l'artère fémorale pour la mesure de la pression artérielle et une canule sur la veine fémorale pour l'administration du produit à tester. L'artère carotide gauche reçoit une canule pour la mise en place du cathéter d'angioplastie, tandis que l'on met en place un cathéter au niveau du ventricule gauche via l'artère fémorale contralatérale.
   Les animaux sont alors héparinisés avec 500 unités U.I. par kg par voie I.V. On procède alors à l'administration du produit à tester ou du placebo à raison, pour le buflomédil, de 10 mg par mn. On administre ensuite de la lidocaïne par voie I.V. à raison de 1 mg/kg sous forme de bolus, puis par perfusion à raison de 1 mg par mn.
   30 mn après la fin de la perfusion, on met en place un ballonnet d'angioplastie de 2,5-3,5 mm choisi en fonction de la taille des artères coronaires dans l'artère coronaire descendante antérieure gauche. Le ballon a été gonflé à une pression de 4.10⁵ Pa pendant 15 mn.
   Chez les chiens traités par le buflomédil on n'a pas observé de fibrillation ventriculaire lors de la reperfusion. Chez les chiens ayant reçu le placebo, on a observé une fibrillation ventriculaire lors de la reperfusion dans 4 cas sur 10.
   On a par ailleurs mesuré les flux sanguins tissulaires au niveau du myocarde. Les résultats mesures pendant l'ischémie dans les régions ischémiées sont reportés sur la Figure annexée.
   Ils mettent en évidence des flux sanguins tissulaires plus élevés chez les chiens traités au buflomédil.
2 - Dans une deuxième série d'essais comparatifs, on a utilisé le même mode opératoire et les mêmes produits (buflomédil chlorhydrate à la dose de 10 mg/kg d'une part et placebo d'autre part) et on a suivi la reperfusion après 15 minutes d'occlusion avec un ballonnet.
   Si on n'a pas observé à la fin de l'occlusion de différence entre les deux groupes en ce qui concerne la fraction d'éjection et la perfusion sanguine transmurale, en revanche 30 minutes après la reperfusion, la fraction d'éjection pour le groupe ayant reçu le buflomédil s'élevait à 89 % de la valeur normale du groupe alors que cette fraction d'éjection s'élevait à 69 % de la normale pour le groupe placebo. Cette différence avec le buflomédil s'est maintenue 60 minutes après la reperfusion ( 95 % avec le buflomédil et 74 % avec le placebo au bout de 60 minutes). Elle est due pour partie à un flux accru pendant la reperfusion et à une diminution de la zone de dysfonctionnement.
   Au plan systémique, on observe donc une augmentation de la fraction d'éjection et ceci met en évidence une amélioration des performances globales du myocarde.
   Par ailleurs, au plan myocardique régional, on observe une diminution de la surface du myocarde rendu ischémique présentant une baisse de la fonction contractile. L'amélioration de la fonction contractile régionale du myocarde au moment de la reperfusion s'accompagne d'une légère augmentation de la perfusion au sein de cette même zone.

## Revendications

1. Utilisation du buflomédil et de ses sels d'addition avec des acides pharmaceutiquement acceptables pour la fabrication d'un médicament pour la protection du myocarde lors d'une angioplastie des coronaires.

2. Utilisation selon la revendication 1 pour la fabication d'un médicament sous forme administrable par voie intraveineuse.

## Claims

1. Use of buflomedil and of the addition salts thereof with pharmaceutically acceptable acids for the preparation of a pharmaceutical composition for protecting the myocardium during coronary angioplasty.

2. Use according to claim 1 for the preparation of a pharmaceutical composition in a form which may be administered intravenously.

## Patentansprüche

1. Verwendung von Buflomedil und seiner Additionssalze mit pharmazeutisch annehmbaren Säuren zur Herstellung eines Arzneimittels zum Schutz des Myokards bei einer Koronar-Angioplastie.

2. Verwendung nach Anspruch 1 zur Herstellung eines Arzneimittels in Form eines auf intravenösem Wege zu verabreichenden Arzneimittels.
